(19)

Europäisches
Patentamt
European
Patent Office
Office européen
des brevets

(11) **EP 4 424 232 A1**

(12) **EUROPEAN PATENT APPLICATION**
published in accordance with Art. 153(4) EPC

(43) Date of publication:
**04.09.2024 Bulletin 2024/36**

(21) Application number: **23740471.0**

(22) Date of filing: **12.01.2023**

(51) International Patent Classification (IPC):
*A61B 5/0205* (2006.01)   *A61B 5/024* (2006.01)
*A61B 5/11* (2006.01)   *A61B 5/00* (2006.01)
*A61B 5/28* (2021.01)   *A61B 5/053* (2021.01)
*G16H 20/30* (2018.01)

(52) Cooperative Patent Classification (CPC):
**A61B 5/00; A61B 5/0205; A61B 5/024;
A61B 5/053; A61B 5/11; A61B 5/28; G16H 20/30**

(86) International application number:
**PCT/KR2023/000577**

(87) International publication number:
**WO 2023/136628 (20.07.2023 Gazette 2023/29)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **13.01.2022 KR 20220005145**

(71) Applicant: Samsung Electronics Co., Ltd.
**Suwon-si, Gyeonggi-do 16677 (KR)**

(72) Inventors:
• **SLYUSARENKO, Kostyantyn**
  **Kyiv, 01032 (UA)**
• **FEDORIN, Illia**
  **Kyiv, 01032 (UA)**
• **KRASNOSHCHOK, Illia**
  **Kyiv, 01032 (UA)**
• **POHRIBNYI, Vitalii**
  **Kyiv, 01032 (UA)**

(74) Representative: **Gulde & Partner**
**Patent- und Rechtsanwaltskanzlei mbB**
**Wallstraße 58/59**
**10179 Berlin (DE)**

(54) **ELECTRONIC DEVICE FOR DETERMINING VITAL SIGN OF USER AND OPERATION METHOD THEREOF**

(57)   Provided is an electronic device that determines a vital sign while a user is active. The electronic device comprises: a first sensor for sensing a vital sign of a user; a second sensor for sensing activity of the user; and at least one processor connected to the first sensor and the second sensor. The at least one processor may be configured to: identify a vital sign determined through a first signal acquired through the first sensor; normalize the vital sign to correspond to the activity characteristics of a user on the basis of the vital sign determined through the first signal and the activity level of the user determined using a second signal acquired through the second sensor; and merge the vital sign determined through the first signal and the normalized vital sign so as to determine a merged vital sign.

FIG. 3

Description

## TECHNICAL FIELD

The disclosure relates to a device and method for determining or modifying a user's vital sign during the user's activity.

## BACKGROUND ART

As interest in health increases, various types of biometric information detection devices for measuring various biometric signals in daily life to enhance convenience have been developed.

Such a device is provided in the form of a wearable device that may be worn on the user to be able to directly measure her biometric condition and is utilized as a smart healthcare system that provides health-related services in association with an individual health information database to provide health-related services and transfers diagnosis/prescription results to the patient.

A need exists for technology for accurately measuring biometric signals of a wearer of a wearable device when the wearer does intensive exercise.

## DETAILED DESCRIPTION OF THE INVENTION

## TECHNICAL PROBLEM

According to an embodiment of the disclosure, an electronic device may accurately measure the biometric signals of the wearer of the electronic device when the wearer does intensive exercise.

According to an embodiment of the disclosure, an electronic device may predict the vital sign of the wearer of the electronic device and provide information helping the user working out.

## TECHNICAL SOLUTION

According to an embodiment of the disclosure are not limited to the foregoing objectives, and other objectives would readily be appreciated by a skilled artisan from the following detailed description taken in conjunction with the accompanying drawings.

According to an embodiment, an electronic device determining a vital sign while a user does an activity comprises a first sensor for detecting the user's vital sign, second sensor for detecting the user's activity, and at least one processor connected to the first sensor and the second sensor. The at least one processor may identify a vital sign determined through a first signal obtained through the first sensor, normalize a vital sign to correspond to the user's activity characteristic based on the vital sign determined through the first signal and the user's activity level determined through a second signal obtained through the second sensor, and determine a vital sign by merging the vital sign determined through the first signal and the normalized vital sign.

According to an embodiment, the at least one processor may identify whether a quality value of the vital sign determined through the first signal is a threshold or more and identify a first vital sign of which the quality value is the threshold or more, of the vital sign determined through the first signal.

According to an embodiment, the at least one processor may identify a normalized vital sign to be used instead of a second vital sign of which the quality value is less than the threshold, of the vital sign determined through the first signal and determine the vital sign in real-time by merging the identified first vital sign and the identified normalized vital sign.

According to ans embodiment, a method for operating an electronic device determining a vital sign while a user does an activity comprises identifying a vital sign determined through a first signal obtained through the first sensor, normalizing a vital sign to correspond to the user's activity characteristic based on the vital sign determined through the first signal and the user's activity level determined through a second signal obtained through the second sensor, and determining a vital sign by merging the vital sign determined through the first signal and the normalized vital sign.

## ADVANTAGEOUS EFFECTS

According to an embodiment of the disclosure, it is possible to measure the biometric signals of the wearer of the electronic device when doing intensive exercise and accurately determine or predict the wearer's vital sign based on the measured biometric signals.

Other aspects, advantages, and salient features of the disclosure will become apparent to those skilled in the art from the following detailed description, which, taken in conjunction with the annexed drawings, discloses various embodiments of the disclosure.

## BRIEF DESCRIPTION OF DRAWINGS

The above and other aspects, features, and advantages of certain embodiments of the disclosure will be more apparent from the following description taken in conjunction with the accompanying drawings, in which:

FIG. 1 is a block diagram illustrating an electronic device in a network environment according to an embodiment of the disclosure;

FIG. 2 is a view illustrating a process for setting a fitness training program per user according to an embodiment of the disclosure.

FIG. 3 is a view illustrating operations of a wearable device according to an embodiment of the disclosure;

FIG. 4 is a view illustrating operations of a system for monitoring and predicting a biometric signal according to an embodiment of the disclosure;

FIG. 5 is a view illustrating operations of a wearable device including a biometric sensor and an activity sensor according to an embodiment of the disclosure;

FIG. 6 is a view illustrating a direct measurement method of a wearable device according to an embodiment of the disclosure;

FIG. 7 is a view illustrating a heart rate (HR) trend calculation method of a wearable device according to an embodiment of the disclosure;

FIG. 8A is a view illustrating a normalization process of a wearable device according to an embodiment of the disclosure;

FIG. 8B is a view illustrating a normalization of a heart rate (HR) pattern according to an embodiment of the disclosure;

FIG. 9A is a view illustrating a process for modifying a heart rate (HR) by a wearable device according to an embodiment of the disclosure;

FIG. 9B is a view illustrating a modified heart rate (HR) according to an embodiment of the disclosure;

FIG. 10 is a view illustrating a heart rate (HR) prediction process of a wearable device according to an embodiment of the disclosure;

FIG. 11A is a view illustrating an activity defined fitness training according to an embodiment of the disclosure; and

FIG. 11B is a view illustrating a heart rate (HR) defined fitness training according to an embodiment of the disclosure.

Throughout the drawings, it should be noted that like reference numbers are used to depict the same or similar elements, features, and structures.

## MODE FOR CARRYING OUT THE INVENTION

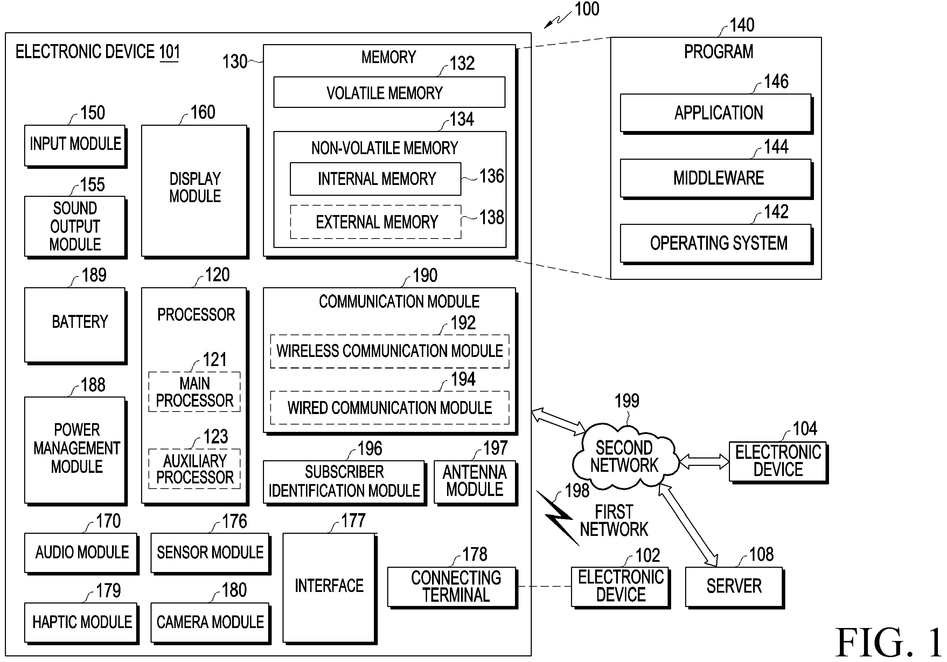

FIG. 1 is a block diagram illustrating an electronic device in a network environment according to an embodiment of the disclosure.

Referring to FIG. 1, an electronic device 101 in a network environment 100 may communicate with at least one of an electronic device 102 via a first network 198 (e.g., a short-range wireless communication network), or an electronic device 104 or a server 108 via a second network 199 (e.g., a long-range wireless communication network). According to an embodiment, the electronic device 101 may communicate with the electronic device 104 via the server 108. According to an embodiment, the electronic device 101 may include a processor 120, a memory 130, an input module 150, a sound output module 155, a display module 160, an audio module 170, a sensor module 176, an interface 177, a connecting terminal 178, a haptic module 179, a camera module 180, a power management module 188, a battery 189, a communication module 190, a subscriber identification module (SIM) 196, or an antenna module 197. In some embodiments, at least one (e.g., the connecting terminal 178) of the components may be omitted from the electronic device 101, or one or more other components may be added in the electronic device 101. According to an embodiment, some (e.g., the sensor module 176, the camera module 180, or the antenna module 197) of the components may be integrated into a single component (e.g., the display module 160).

The processor 120 may execute, for example, software (e.g., a program 140) to control at least one other component (e.g., a hardware or software component) of the electronic device 101 coupled with the processor 120, and may perform various data processing or computation. According to one embodiment, as at least part of the data processing or computation, the processor 120 may store a command or data received from another component (e.g., the sensor module 176 or the communication module 190) in a volatile memory 132, process the command or the data stored in the volatile memory 132, and store resulting data in a non-volatile memory 134. According to an embodiment, the processor 120 may include a main processor 121 (e.g., a central processing unit (CPU) or an application processor (AP)), or an auxiliary processor 123 (e.g., a graphics processing unit (GPU), a neural processing unit (NPU), an image signal processor (ISP), a sensor hub processor, or a communication processor (CP)) that is operable independently from, or in conjunction with, the main processor 121. For example, when the electronic device 101 includes the main processor 121 and the auxiliary processor 123, the auxiliary processor 123 may be configured to use lower power than the main processor 121 or to be specified for a designated function. The auxiliary processor 123 may be implemented as separate from, or as part of the main processor 121.

The auxiliary processor 123 may control at least some of functions or states related to at least one component (e.g., the display module 160, the sensor module 176, or the communication module 190) among the components of the electronic device 101, instead of the main processor 121 while the main processor 121 is in an inactive (e.g., sleep) state, or together with the main processor 121 while the main processor 121 is in an active state (e.g., executing an application). According to an embodiment, the auxiliary processor 123 (e.g., an image signal processor or a communication processor) may be implemented as part of another component (e.g., the camera module 180 or the communication module 190) functionally related to the auxiliary processor 123. According to an embodiment, the auxiliary processor 123 (e.g., the neural processing unit) may include a hardware structure specified for artificial intelligence model processing. The artificial intelligence model may be generated via machine learning. Such learning may be performed, e.g., by the electronic device 101 where the artificial intelligence is performed or via a separate server (e.g., the server 108). Learning algorithms may include, but are not limited to, e.g., supervised learning, unsupervised learning, semi-supervised learning, or reinforcement learning. The artificial intelligence model may include a plurality of artificial neural network layers. The artificial neural network may be a deep neural network (DNN), a convolutional neural network (CNN), a recurrent neural network (RNN), a restricted Boltzmann machine (RBM), a deep belief network (DBN), a bidirectional recurrent deep neural network (BRDNN), deep Q-network or a combination of two or more thereof but is not limited thereto. The artificial intelligence model may, additionally or alternatively, include a software structure other than the hardware structure.

The memory 130 may store various data used by at least one component (e.g., the processor 120 or the sensor module 176) of the electronic device 101. The various data may include, for example, software (e.g., the program 140) and input data or output data for a command related thereto. The memory 130 may include the volatile memory 132 or the non-volatile memory 134.

The program 140 may be stored in the memory 130 as software, and may include, for example, an operating system (OS) 142, middleware 144, or an application 146.

The input module 150 may receive a command or data to be used by other component (e.g., the processor 120) of the electronic device 101, from the outside (e.g., a user) of the electronic device 101. The input module 150 may include, for example, a microphone, a mouse, a keyboard, keys (e.g., buttons), or a digital pen (e.g., a stylus pen).

The sound output module 155 may output sound signals to the outside of the electronic device 101. The sound output module 155 may include, for example, a speaker or a receiver. The speaker may be used for general purposes, such as playing multimedia or playing record. The receiver may be used for receiving incoming calls. According to an embodiment, the receiver may be implemented as separate from, or as part of the speaker.

The display module 160 may visually provide information to the outside (e.g., a user) of the electronic device 101. The display 160 may include, for example, a display, a hologram device, or a projector and control circuitry to control a corresponding one of the display, hologram device, and projector. According to an embodiment, the display 160 may include a touch sensor configured to detect a touch, or a pressure sensor configured to measure the intensity of a force generated by the touch.

The audio module 170 may convert a sound into an electrical signal and vice versa. According to an embodiment, the audio module 170 may obtain the sound via the input module 150, or output the sound via the sound output module 155 or a headphone of an external electronic device (e.g., an electronic device 102) directly (e.g., wiredly) or wirelessly coupled with the electronic device 101.

The sensor module 176 may detect an operational state (e.g., power or temperature) of the electronic device 101 or an environmental state (e.g., a state of a user) external to the electronic device 101, and then generate an electrical signal or data value corresponding to the detected state. According to an embodiment, the sensor module 176 may include, for example, a gesture sensor, a gyro sensor, an atmospheric pressure sensor, a magnetic sensor, an accelerometer, a grip sensor, a proximity sensor, a color sensor, an infrared (IR) sensor, a biometric sensor, a temperature sensor, a humidity sensor, or an illuminance sensor.

The interface 177 may support one or more specified protocols to be used for the electronic device 101 to be coupled with the external electronic device (e.g., the electronic device 102) directly (e.g., wiredly) or wirelessly. According to an embodiment, the interface 177 may include, for example, a high definition multimedia interface (HDMI), a universal serial bus (USB) interface, a secure digital (SD) card interface, or an audio interface.

A connecting terminal 178 may include a connector via which the electronic device 101 may be physically connected with the external electronic device (e.g., the electronic device 102). According to an embodiment, the connecting terminal 178 may include, for example, a HDMI connector, a USB connector, a SD card connector, or an audio connector (e.g., a headphone connector).

The haptic module 179 may convert an electrical signal into a mechanical stimulus (e.g., a vibration or motion) or electrical stimulus which may be recognized by a user via his tactile sensation or kinesthetic sensation. According to an embodiment, the haptic module 179 may include, for example, a motor, a piezoelectric element, or an electric stimulator.

The camera module 180 may capture a still image or moving images. According to an embodiment, the camera module 180 may include one or more lenses, image sensors, image signal processors, or flashes.

The power management module 188 may manage power supplied to the electronic device 101. According to one embodiment, the power management module 188 may be implemented as at least part of, for example, a power management integrated circuit (PMIC).

The battery 189 may supply power to at least one component of the electronic device 101. According to an embodiment, the battery 189 may include, for example, a primary cell which is not rechargeable, a secondary cell which is rechargeable, or a fuel cell.

The communication module 190 may support establishing a direct (e.g., wired) communication channel or a wireless communication channel between the electronic device 101 and the external electronic device (e.g., the electronic device 102, the electronic device 104, or the server 108) and performing communication via the established communication channel. The communication module 190 may include one or more communication processors that are operable independently from the processor 120 (e.g., the application processor (AP)) and supports a direct (e.g., wired) communication or a wireless communication. According to an embodiment, the communication module 190 may include a wireless communication module 192 (e.g., a cellular communication module, a short-range wireless communication module, or a global navigation satellite system (GNSS) communication module) or a wired communication module 194 (e.g., a local area network (LAN) communication module or a power line communication (PLC) module). A corresponding one of these communication modules may communicate with the external electronic device 104 via a first network 198 (e.g., a short-range communication network, such as Bluetooth™, wireless-fidelity (Wi-Fi) direct, or infrared data association (IrDA)) or a second network 199 (e.g., a long-range communication network, such as a legacy cellular network, a fifth generation (5G) network, a next-generation communication network, the Internet, or a computer network (e.g., local area network (LAN) or wide area network (WAN)). These various types of communication modules may be implemented as a single component (e.g., a single chip), or may be implemented as multi components (e.g., multi chips) separate from each other. The wireless communication module 192 may identify or authenticate the electronic device 101 in a communication network, such as the first network 198 or the second network 199, using subscriber information (e.g., international mobile subscriber identity (IMSI)) stored in the subscriber identification module 196.

The wireless communication module 192 may support a 5G network, after a fourth generation (4G) network, and next-generation communication technology, e.g., new radio (NR) access technology. The NR access technology may support enhanced mobile broadband (eMBB), massive machine type communications (mMTC), or ultra-reliable and low-latency communications (URLLC). The wireless communication module 192 may support a high-frequency band (e.g., the millimeter wave (mmWave) band) to achieve, e.g., a high data transmission rate. The wireless communication module 192 may support various technologies for securing performance on a high-frequency band, such as, e.g., beamforming, massive multiple-input and multiple-output (massive MIMO), full dimensional MIMO (FD-MIMO), array antenna, analog beam-forming, or large scale antenna. The wireless communication module 192 may support various requirements specified in the electronic device 101, an external electronic device (e.g., the electronic device 104), or a network system (e.g., the second network 199). According to an embodiment, the wireless communication module 192 may support a peak data rate (e.g., 20Gbps or more) for implementing eMBB, loss coverage (e.g., 164dB or less) for implementing mMTC, or U-plane latency (e.g., 0.5ms or less for each of downlink (DL) and uplink (UL), or a round trip of 1ms or less) for implementing URLLC.

The antenna module 197 may transmit or receive a signal or power to or from the outside (e.g., the external electronic device). According to an embodiment, the antenna module 197 may include one antenna including a radiator formed of a conductor or conductive pattern formed on a substrate (e.g., a printed circuit board (PCB)). According to an embodiment, the antenna module 197 may include a plurality of antennas (e.g., an antenna array). In this case, at least one antenna appropriate for a communication scheme used in a communication network, such as the first network 198 or the second network 199, may be selected from the plurality of antennas by, e.g., the communication module 190. The signal or the power may then be transmitted or received between the communication module 190 and the external electronic device via the selected at least one antenna. According to an embodiment, other parts (e.g., radio frequency integrated circuit (RFIC)) than the radiator may be further formed as part of the antenna module 197.

According to various embodiments, the antenna module 197 may form a mmWave antenna module. According to an embodiment, the mmWave antenna module may include a printed circuit board, a RFIC disposed on a first surface (e.g., the bottom surface) of the printed circuit board, or adjacent to the first surface and capable of supporting a designated high-frequency band (e.g., the mmWave band), and a plurality of antennas (e.g., array antennas) disposed on a second surface (e.g., the top or a side surface) of the printed circuit board, or adjacent to the second surface and capable of transmitting or receiving signals of the designated high-frequency band.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, commands or data may be transmitted or received between the electronic device 101 and the external electronic device 104 via the server 108 coupled with the second network 199. The external electronic devices 102 or 104 each may be a device of the same or a different type from the electronic device 101. According to an embodiment, all or some of operations to be executed at the electronic device 101 may be executed at one or more of the external electronic devices 102, 104, or 108. For example, if the electronic device 101 should perform a function or a service automatically, or in response to a request from a user or another device, the electronic device 101, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 101. The electronic device 101 may provide the outcome, with or without further processing of the outcome, as at least part of a reply to the request. To that end, a cloud computing, distributed computing, mobile edge computing (MEC), or client-server computing technology may be used, for example. The electronic device 101 may provide ultra low-latency services using, e.g., distributed computing or mobile edge computing. In another embodiment, the external electronic device 104 may include an Internet-of-things (IoT) device. The server 108 may be an intelligent server using machine learning and/or a neural network. According to an embodiment, the external electronic device 104 or the server 108 may be included in the second network 199. The electronic device 101 may be applied to intelligent services (e.g., smart home, smart city, smart car, or healthcare) based on 5G communication technology or IoT-related technology.

The electronic device according to various embodiments of the disclosure may be one of various types of electronic devices. The electronic devices may include, for example, a portable communication device (e.g., a smart phone), a computer device, a portable multimedia device, a portable medical device, a camera, a wearable device, or a home appliance. According to an embodiment of the disclosure, the electronic devices are not limited to those described above.

It should be appreciated that various embodiments of the present disclosure and the terms used therein are not intended to limit the technological features set forth herein to particular embodiments and include various changes, equivalents, or replacements for a corresponding embodiment. With regard to the description of the drawings, similar reference numerals may be used to refer to similar or related elements. It is to be understood that a singular form of a noun corresponding to an item may include one or more of the things, unless the relevant context clearly indicates otherwise. As used herein, each of such phrases as "A or B," "at least one of A and B," "at least one of A or B," "A, B, or C," "at least one of A, B, and C," and "at least one of A, B, or C," may include all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with," "coupled to," "connected with," or "connected to" another element (e.g., a second element), it means that the element may be coupled with the other element directly (e.g., wiredly), wirelessly, or via a third element.

As used herein, the term "module" may include a unit implemented in hardware, software, or firmware, and may interchangeably be used with other terms, for example, "logic," "logic block," "part," or "circuitry". A module may be a single integral component, or a minimum unit or part thereof, adapted to perform one or more functions. For example, according to an embodiment, the module may be implemented in a form of an application-specific integrated circuit (ASIC).

Various embodiments as set forth herein may be implemented as software (e.g., the program 140) including one or more instructions that are stored in a storage medium (e.g., internal memory 136 or external memory 138) that is readable by a machine (e.g., the electronic device 101). For example, a processor (e.g., the processor 120) of the machine (e.g., the electronic device 101) may invoke at least one of the one or more instructions stored in the storage medium, and execute it, with or without using one or more other components under the control of the processor. This allows the machine to be operated to perform at least one function according to the at least one instruction invoked. The one or more instructions may include a code generated by a complier or a code executable by an interpreter. The machine-readable storage medium may be provided in the form of a non-transitory storage medium. Wherein, the term "non-transitory" simply means that the storage medium is a tangible device, and does not include a signal (e.g., an electromagnetic wave), but this term does not differentiate between where data is semi-permanently stored in the storage medium and where the data is temporarily stored in the storage medium.

According to an embodiment, a method according to various embodiments of the disclosure may be included and provided in a computer program product. The computer program products may be traded as commodities between sellers and buyers. The computer program product may be distributed in the form of a machine-readable storage medium (e.g., a compact disc read only memory (CD-ROM)), or be distributed (e.g., downloaded or uploaded) online via an application store (e.g., Play Store™), or between two user devices (e.g., smart phones) directly. If distributed online, at least part of the computer program product may be temporarily generated or at least temporarily stored in the machine-readable storage medium, such as a memory of the manufacturer's server, a server of the application store, or a relay server.

According to various embodiments, each component (e.g., a module or a program) of the above-described components may include a single entity or multiple entities. Some of the plurality of entities may be separately disposed in different components. According to various embodiments, one or more of the above-described components may be omitted, or one or more other components may be added. Alternatively or additionally, a plurality of components (e.g., modules or programs) may be integrated into a single component. In such a case, according to various embodiments, the integrated component may still perform one or more functions of each of the plurality of components in the same or similar manner as they are performed by a corresponding one of the plurality of components before the integration. According to various embodiments, operations performed by the module, the program, or another component may be carried out sequentially, in parallel, repeatedly, or heuristically, or one or more of the operations may be executed in a different order or omitted, or one or more other operations may be added.

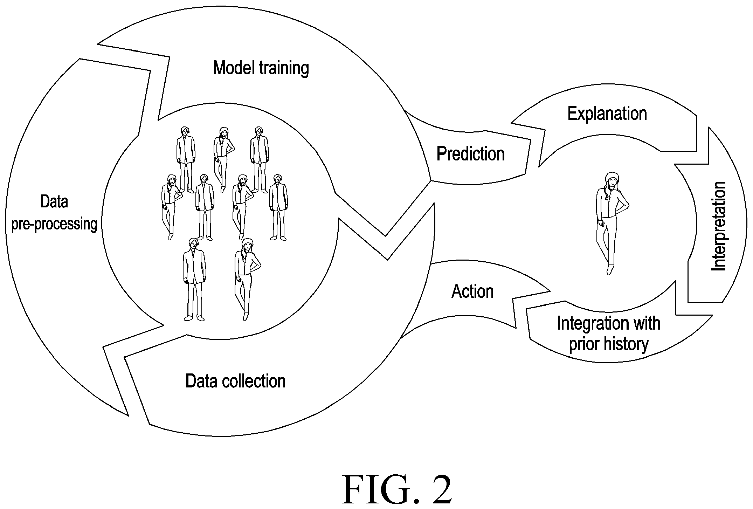

FIG. 2 is a view illustrating a process for setting a fitness training program per user according to an embodiment of the disclosure.

According to various embodiments of the disclosure, since biological reaction differs from person to person, the fitness training program needs to be modified to fit each user to produce an exercise effect. According to various embodiments, the cardiovascular system affects heart rate inertia and slow heart rate changes even with rapid changes in activity, and it is necessary to preset an effective training schedule and consider individual cardiovascular characteristics according to activity.

Referring to FIG. 2, the fitness training program for each user may be performed through data pre-processing, model training, and data collection processes. According to various embodiments, to set a fitness training program for each user, exercise data may be collected for each user, the data may be pre-processed, and fitness model training may be performed. According to various embodiments, the user's exercise data may be collected (or recollected) through prediction, explanation, interpretation, integration with prior history, and action processes on the user's exercise after model training.

According to various embodiments, when the user performs intensive exercise with a wearable device (e.g., a smart watch) worn, noise due to the user's movement is reflected, causing the heart rate, measured through a heart rate sensor in the wearable device, inaccurate. According to various embodiments, the heart rate sensor may include at least one of a photoplethysmogram (PPG) sensor and an electrocardiogram (ECG) sensor. According to various embodiments, the PPG sensor may be periodically exposed to ambient light saturation due to the user's hand motion during intensive exercise. According to various embodiments, the ECG sensor may generate noise due to a change in the electrode contact area during the user's intensive exercise.

According to various embodiments, cardiac output refers to the total amount of blood expelled from the heart and is typically measured in liters per minute. Heart rate refers to how often the heart beats and may be measured per minute. Stroke volume refers to the amount of blood ejected from the heart at each beat, and the cardiac output may be the product of the heart rate and the stroke volume. According to various embodiments of the disclosure, when the exercise intensity of the wearable device user is 50-60% of the maximum heart rate, an increase in cardiac output may be due to an increase in heart rate and stroke volume. According to various embodiments, when the exercise intensity exceeds 60% of the maximum heart rate when the user of the wearable device does exercise, an increase in cardiac output may be entirely due to an increase in heart rate. According to various embodiments, when the user of the wearable device does intensive exercise in which the exercise intensity exceeds 60% of the maximum heart rate, cardiac output may increase due to an increase in heart rate.

According to various embodiments, the heart rate (HR) may be finely measured when the user is calm. According to various embodiments, the wearable device may include at least one of a photoplethysmogram (PPG) sensor, an electrocardiogram (ECG) sensor, and a bioimpedance (BIA) sensor to measure the heart rate. For example, the PPG sensor may be implemented to be included in a smart watch, the ECG sensor may be implemented to be included in a fitness belt, and the BIA sensor may be implemented to be included in a fitness tracker and a smart ring. According to various embodiments, intensive exercise of the wearable device user may have a negative effect on all heart rate sensors. For example, the PPG sensor may expose periodic ambient light saturation due to the user's hand movement, the ECG sensor may generate noise due to a change in electrode contact area due to the user's body transformation, and the BIA sensor may generate noise due to neural impulses (e.g., mussels neural impulses) caused by the user's movement.

According to various embodiments, the electronic device (or wearable device) may detect and predict the heart rate (HR) of the user during exercise considering both the direct heart rate signal measured through the heart rate sensor and the user movement detected through the motion sensor. According to various embodiments, a measurement error generated according to the user's movement may be included in the direct heart rate signal measured by the heart rate sensor.

According to various embodiments, the electronic device (or wearable device) may identify a time duration in which the user moves less based on motion data corresponding to the user's movement and sets the user's movement-heart rate relationship using the heart rate data of the time duration in which the user moves less to thereby enhance the accuracy of heart rate estimation for the user. According to various embodiments, the heart rate data of the time duration in which the user moves less may include fewer measurement errors than the heart rate data in the time duration in which the user moves more.

According to various embodiments, the electronic device (or wearable device) may set (or correct) the heart rate (HR) trend to be user customized using the relationship between the motion data and the heart rate value when the heart rate is well measured for each user (or heart rate value of good quality). According to various embodiments, the heart rate value when the heart rate is well measured (or heart rate value of good quality) may be measured in a time duration in which the user moves less. In the disclosure, setting (or correcting) the heart rate (HR) trend to be user-customized may also be expressed as "normalizing the heart rate trend".

According to various embodiments, the electronic device (or wearable device) may estimate the user's heart rate using the normalized heart rate trend information in the time duration in which the user's heart rate is not well measured due to noise.

According to various embodiments, the electronic device may measure the user's activity intensity in a preset time duration considering the user's movement. According to various embodiments, the electronic device may detect and calculate the heart rate (HR) in real-time during intensive exercise by the user wearing the electronic device and may predict a heart rate (HR) pattern for a given activity pattern. According to various embodiments, the electronic device may predict and calculate an activity pattern for a heart rate (HR) pattern required in the future.

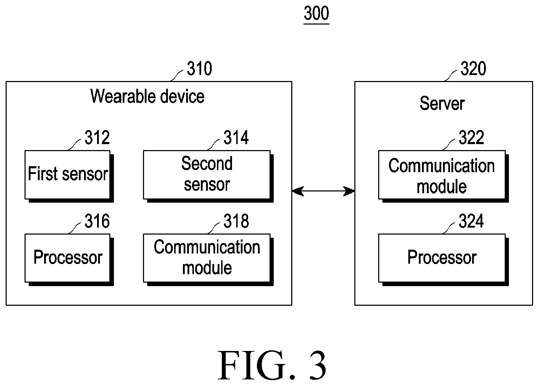

FIG. 3 is a view 300 illustrating operations of a wearable device according to an embodiment of the disclosure.

Referring to FIG. 3, a wearable device 310 may include a first sensor 312, a second sensor 314, a processor 316, and a communication module 318. According to various embodiments, the wearable device 310 may be implemented as the electronic device 101 of FIG. 1. According to various embodiments, the first sensor 312 may be at least one of a photoplethysmogram (PPG) sensor, an electrocardiogram (ECG) sensor, and a bioimpedance (BIA) sensor and may also be referred to as a vital sign measurement sensor. According to various embodiments, the second sensor 314 may be at least one of a gyroscope, an accelerometer, a geomagnetic sensor, a radar sensor, and a video camera and may also be referred to as a motion detection sensor (or motion sensor). According to various embodiments, the server 320 may include a communication module 322 and a processor 324. According to various embodiments, the server 320 may be implemented as the server 108 of FIG. 1.

According to various embodiments, the processor 316 implemented in the wearable device 310 may identify a vital sign determined through a first signal obtained through the first sensor 312. According to various embodiments, the vital sign may be any one of a heart rate, a stress value, a respiration rate, and a lactate value. According to various embodiments, the processor 316 implemented in the wearable device 310 may normalize the vital sign determined through the first signal to be user-customized according to the user's activity level determined based on a second signal obtained through the second sensor 314. According to various embodiments, the activity level may be determined based on at least one of the user's activity type, the user's activity intensity, and the user's activity duration.

According to various embodiments, the processor 316 implemented in the wearable device 310 may identify the time duration in which the user moves less according to the user's activity level determined based on the second signal obtained through the second sensor 314. According to various embodiments, the processor 316 may compare the user's activity level value with a threshold and, when the activity level value is smaller than the threshold, may determine that the user moves less.

According to various embodiments, the processor 316 implemented in the wearable device 310 may identify the vital sign based on the first signal obtained through the first sensor 312 in the time duration in which the user moves less. According to various embodiments, the processor 316 implemented in the wearable device 310 may enhance the accuracy of vital sign estimation for the user by setting the user's movement-vital sign relationship in the time duration in which the user moves less. According to various embodiments, the vital sign in the time duration in which the user moves less may contain fewer measurement errors than the vital sign in the time duration in which the user moves more.

According to various embodiments, the processor 316 implemented in the wearable device 310 may set (or correct) the vital sign trend (e.g., heart rate trend) to be user-customized, using the user's movement-vital sign relationship. In the disclosure, setting (or correcting) the vital sign trend to be customized for each user may also be referred to as "normalizing the vital sign trend."

According to various embodiments, the processor 316 implemented in the wearable device 310 may estimate the user's vital sign using the normalized vital sign trend information even in a duration in which the vital sign is not well measured through the first sensor 312.

According to various embodiments, the processor 316 implemented in the wearable device 310 may modify the vital sign (in real-time) by merging the vital sign determined through the first signal and the vital sign according to the normalized vital sign trend. According to various embodiments, the processor 316 implemented in the wearable device 310 may predict the vital sign for the user's future activity pattern based on the modified vital sign. According to various embodiments, the processor 316 implemented in the wearable device 310 may calculate the user's future activity level pattern for the required vital sign pattern.

According to various embodiments, the processor 316 implemented in the wearable device 310 may calculate or restore the trend of the vital sign based on the activity level, determined through the second signal obtained through the second sensor 314, and machine learning. According to various embodiments, the processor 316 implemented in the wearable device 310 may merge the vital sign determined through the first signal and the vital sign according to the normalized vital sign trend, based on machine learning.

According to various embodiments, the server 320 may transmit/receive information to and from the wearable device 310 through the communication module 322. According to various embodiments, the server 320 may receive, from the wearable device 310, at least one of information regarding the vital sign determined through the first signal obtained through the first sensor 312, information regarding the user's activity level determined through the second signal obtained through the second sensor 314, and information regarding the vital sign modified by merging the vital sign determined through the first signal and the vital sign according to the normalized vital sign trend.

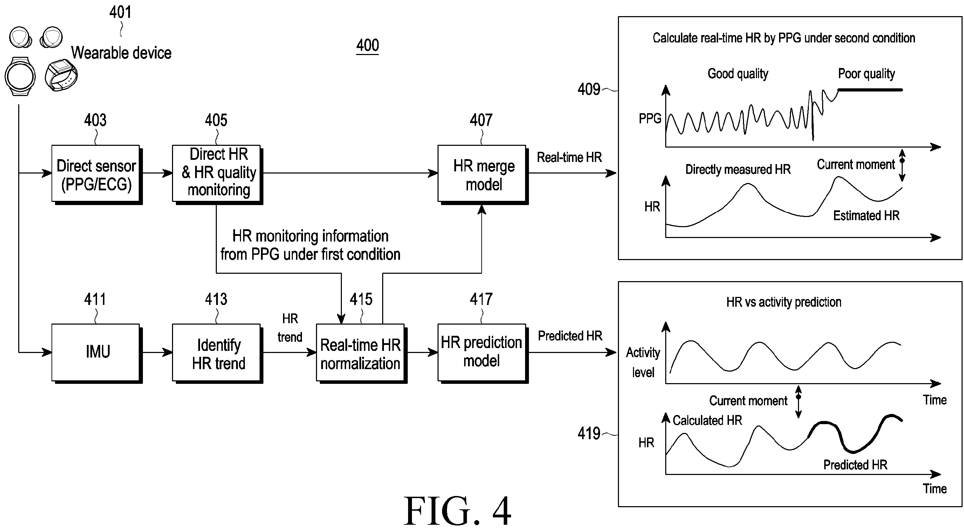

FIG. 4 is a view illustrating operations of a system for monitoring and predicting a biometric signal according to an embodiment of the disclosure.

Referring to FIG. 4, a system 400 for monitoring and predicting a biometric signal may include at least one wearable device 401. The at least one wearable device 401 may include a direct sensor (e.g., a PPG sensor or an ECG sensor) 403 and an inertial measurement unit (IMU) 411. According to various embodiments, the direct sensor 403 may measure a biometric signal (e.g., heart rate (HR)) of the user wearing at least one wearable device 401. According to various embodiments, the biometric signal (e.g., heart rate (HR)) measured by the direct sensor 403 may include noise due to the user's movement. According to various embodiments, the IMU 411 may measure the inertia of the at least one wearable device 401 to measure the angle at which the at least one wearable device 401 is tilted. According to various embodiments, the IMU 411 may be configured as at least one of a gyroscope, an accelerometer, and a geomagnetic sensor. According to various embodiments, the direct sensor 403 may correspond to the first sensor 312 of FIG. 3, and the IMU 411 may correspond to the second sensor 314 of FIG. 3.

According to various embodiments, the at least one wearable device 401 may measure the heart rate (HR) of the user wearing the at least one wearable device 401 through the direct sensor 403 and may monitor heart rate (HR) quality in operation 405. According to various embodiments, the at least one wearable device 401 may determine the heart rate (HR) quality based on the degree to which noise and/or errors are included in the heart rate (HR) measured by the direct sensor 403. According to various embodiments, the at least one wearable device 401 may identify the heart rate (HR) trend of the user wearing the at least one wearable device 401 based on the signal obtained through the IMU 411 in operation 413.

According to various embodiments, the at least one wearable device 401 may perform user-customized heart rate (HR) normalization in real-time, based on the heart rate (HR) trend identified through the IMU 411 and the monitoring information about the heart rate (HR) measured by the direct sensor 403 under a preset first condition. The heart rate may be normalized to be user-customized based on the relationship between the heart rate (HR) measured by the direct sensor 403 under the first condition and the user's movement (or activity level) measured through the IMU 411. According to various embodiments, the heart rate trend according to activity level may differ per user, and the user's heart rate may be estimated or predicted more accurately by normalizing the heart rate according to the activity level per user.

According to various embodiments, the preset first condition may mean that the direct sensor 403 detects a heart rate (HR) of good quality (e.g., when the noise included in the heart rate measurement result is a threshold or less). According to various embodiments, the preset first condition may mean that the quality value of heart rate (HR) measured by the direct sensor 403 is the preset threshold or more.

According to various embodiments, the at least one wearable device 401 may perform heart rate (HR) merge modeling based on the monitoring information (heart rate information and quality information) about the heart rate (HR) measured by the direct sensor 403 and the information regarding the heart rate (HR) normalized to be customized according to the user's movement data (or activity level) in step 415 (407). According to various embodiments, the heart rate (HR) merge modeling may mean a modeling scheme using information regarding the heart rate (HR) normalized to be customized according to the user's movement data (or activity level) to accurately estimate the heart rate at the time when the quality of the heart rate (HR) measured by the direct sensor 403 is not good. For example, the heart rate (HR) merge modeling may mean a scheme of determining the heart rate in real-time by merging the heart rate of good quality, measured by the direct sensor 403, and the heart rate estimated according to the information regarding the normalized heart rate (HR).

According to various embodiments, the at least one wearable device 401 may determine the real-time heart rate (HR) based on the result of heart rate (HR) merge modeling. The at least one wearable device 401 may determine (or calculate) the real-time heart rate (HR) by the direct sensor 403 under a preset second condition considering the real-time heart rate (HR) determined based on the heart rate (HR) merge modeling result. According to various embodiments, the preset second condition may mean that the direct sensor 403 detects a heart rate (HR) of poor quality (e.g.., when the noise in the heart rate measurement result exceeds a threshold). According to various embodiments, the preset second condition may mean that the quality value of heart rate (HR) measured by the direct sensor 403 is less than the preset threshold.

According to various embodiments, the at least one wearable device 401 may perform heart rate (HR) prediction modeling based on the information regarding the normalized heart rate (HR) in operation 417. According to various embodiments, the at least one wearable device 401 may identify the activity level of the user wearing the at least one wearable device 401 through the IMU 411 to perform activity prediction (or activity forecasting) on the user in operation 419. According to various embodiments, the at least one wearable device 401 may predict the future heart rate (HR) based on the user's activity prediction (or activity forecasting) according to the user's activity level in operation 419.

According to various embodiments, the at least one wearable device 401 may determine or identify the user's heart rate (HR) with minimized noise by merging the heart rate (HR) information measured by the direct sensor 403 and the user's activity level-based heart rate (HR) trend information. According to various embodiments, the at least one wearable device 401 may correct the heart rate (HR) in real-time even in the case of poor signal quality, predict the heart rate (HR) pattern for a given activity pattern and modify the user's future activity pattern for the required heart rate (HR) pattern.

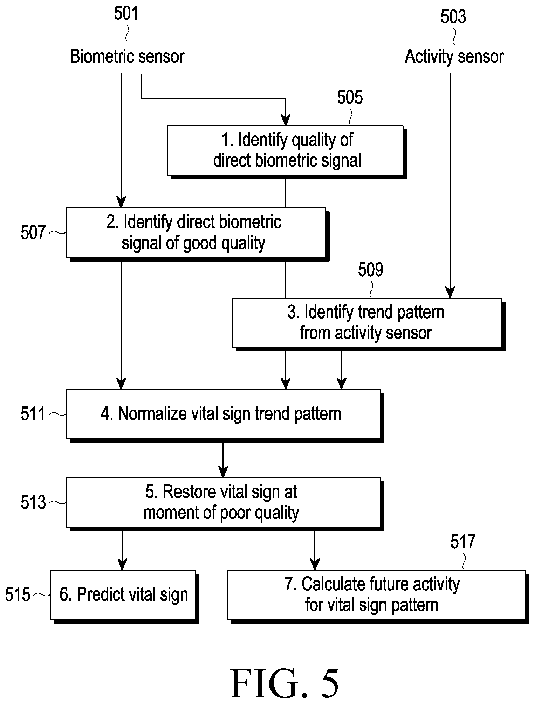

FIG. 5 is a view illustrating operations of a wearable device including a biometric sensor and an activity sensor according to an embodiment of the disclosure.

Referring to FIG. 5, the wearable device may include a biometric sensor 501 and an activity sensor 503. According to various embodiments, the wearable device may calculate the user's vital sign during the activity of the user wearing the wearable device. According to various embodiments, upon failing to calculate the user's vital sign, the wearable device may store the vital sign and predict the heart rate pattern for a future moment for a known future exercise type.

According to various embodiments, the biometric sensor 501 may be the first sensor 312 of FIG. 3 or the direct sensor 403 of FIG. 4. According to various embodiments, the biometric sensor 501 may include at least one of a photoplethysmogram (PPG) sensor, an electrocardiogram (ECG) sensor, and a bioimpedance (BIA) sensor to measure the heart rate.

According to various embodiments, the activity sensor 503 may be the second sensor 314 of FIG. 3 or the IMU 411 of FIG. 4. According to various embodiments, the activity sensor 503 means an inertial measurement unit (IMU), and may include at least one of a gyroscope, an accelerometer, and a geomagnetic sensor.

Referring to FIG. 5, in step 1, the wearable device may identify the quality of the direct biometric signal obtained through the biometric sensor 501 to calculate the user's vital sign in operation 505. According to various embodiments, the user's vital sign may be a heart rate calculated directly from a photoplethysmogram (PPG) signal, a respiration rate calculated directly from a nasal thermistor, or a blood pressure calculated directly from a cuff tonometer. In step 2, when the signal quality of the direct biometric signal meets a set threshold condition, the wearable device may calculate the vital sign from the direct biometric signal in operation 507.

In step 3, the wearable device may identify or calculate the trend pattern of the vital sign based on the activity signal obtained from the activity sensor 503 in operation 509. According to various embodiments, the activity signal may be, when the activity intensity is IMU power, the measurement by the IMU sensor, the activity intensity calculated from the video by activity recognition, the measurement by a motion detection laser/radar/lidar sensor, or global positioning system (GPS) positioning value.

In step 4, the wearable device may real-time normalize the trend pattern of the vital sign to be user-customized based on the vital sign obtained from the biometric signal 501 and the trend pattern of the vital sign obtained from the activity sensor 503 in operation 511.

In step 5, when unable to obtain or calculate the vital sign (or when the direct biometric signal is of poor quality or when the quality of the direct biometric signal is a set threshold or less), the wearable device may real-time restore the vital sign based on the vital sign trend pattern normalized to be user-customized in operation 513.

In step 6, the wearable device may predict a future vital sign for a given future activity pattern considering the vital sign restored in step 5 in operation 515. In step 7, the wearable device may calculate the future activity pattern for the required vital sign pattern considering the vital sign restored in step 5 in operation 517.

According to various embodiments, when the wearable device is a smart watch, the smart watch may monitor the heart rate of the smart watch user while the user runs, based on the photoplethysmogram (PPG) signal and accelerometer (ACC) signal using machine learning. According to various embodiments, the smart watch may predict the future heart rate pattern for the user's given future activity pattern and calculate the future activity pattern for the required heart rate pattern.

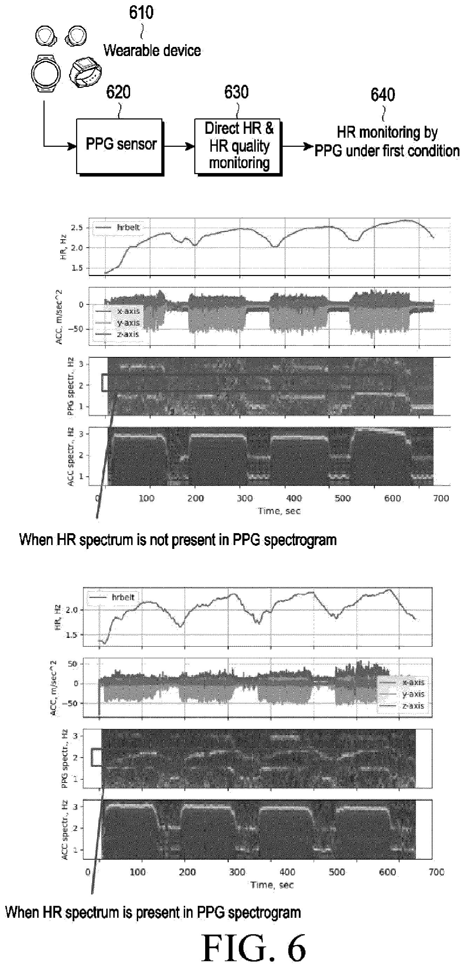

FIG. 6 is a view illustrating a direct measurement method of a wearable device according to an embodiment of the disclosure.

Referring to FIG. 6, at least one wearable device 610 may measure a direct biometric signal using a PPG sensor 620 and monitor the heart rate (HR) and quality of the heart rate (HR) from the measured biometric signal in operation 630. According to various embodiments, the at least one wearable device 610 may measure a direct biometric signal using the PPG sensor 620 in a preset first condition (e.g., when the quality of the biometric signal measured using the PPG sensor 620 is a preset threshold or more or when the quality of the biometric signal measured using the PPG sensor 620 according to a set criterion is determined to be good) and monitor the heart rate (HR) from the measured biometric signal in operation 640.

According to various embodiments, when the heart rate (HR) spectrum is not present in the PPG spectrogram (or when the HR pattern is not detected in the PPG spectrum), the at least one wearable device 610 may determine that the quality of the biometric signal measured using the PPG sensor 620 is poor. According to various embodiments, when the heart rate (HR) spectrum is present in the PPG spectrogram (or when the HR pattern is clearly detected in the PPG spectrum), the at least one wearable device 610 may determine that the quality of the biometric signal measured using the PPG sensor 620 is good.

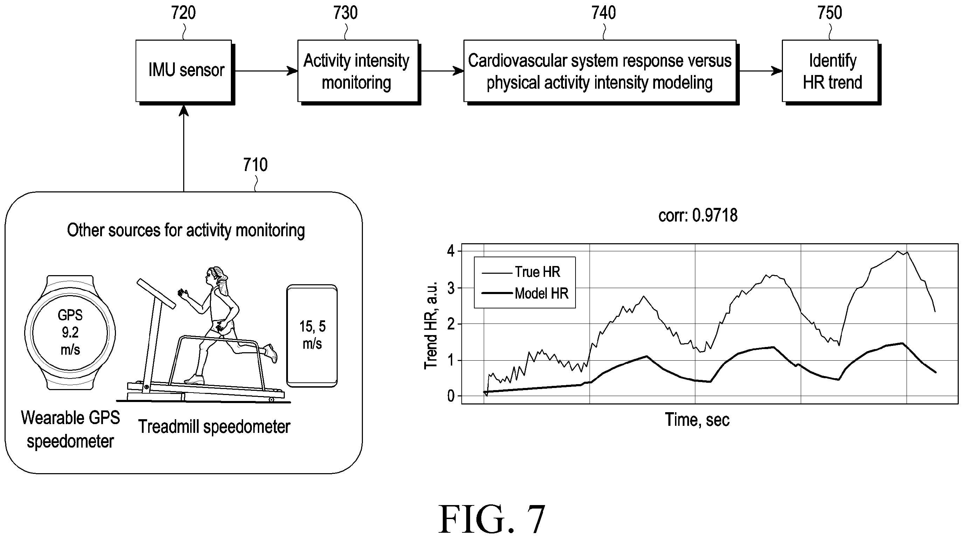

FIG. 7 is a view illustrating a heart rate (HR) trend calculation method of a wearable device according to an embodiment of the disclosure.

Referring to FIG. 7, a wearable GPS speedometer or a treadmill speedometer may exist as a source for monitoring the wearable device user's activity in operation 710. According to various embodiments, the wearable device may monitor the user's activity intensity by an IMU sensor 720 in operation 730. According to various embodiments, the wearable device may perform modeling of the user's cardiovascular system response versus physical activity intensity based on the monitored activity intensity in operation 740. According to various embodiments, the wearable device may identify a heart rate (HR) trend based on the result of modeling the cardiovascular system response versus physical activity intensity in operation 750. According to various embodiments, the wearable device may identify the trend of the user's vital sign (e.g., stress or respiration rate) based on the modeling result of the cardiovascular system response versus physical activity intensity.

According to various embodiments, the wearable device may identify a correlation between the heart rate (HR) trend identified based on the modeling result and the actual heart rate (HR).

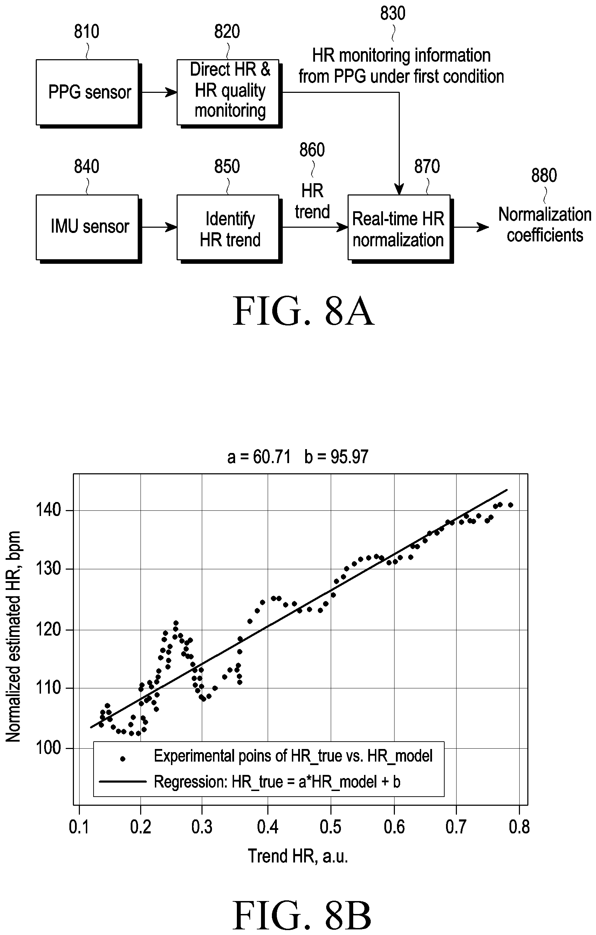

FIG. 8A is a view illustrating a normalization process of a wearable device according to an embodiment of the disclosure. FIG. 8B is a view illustrating a normalizing a heart rate (HR) pattern according to an embodiment of the disclosure.

Referring to FIG. 8A, the wearable device may monitor the heart rate (HR) and the quality of the heart rate (HR) based on the biometric signal measured using the PPG sensor 810 in operation 820. According to various embodiments, the wearable device may identify the heart rate (HR) trend based on the activity intensity (or activity level) measured using the IMU sensor 840 in operation 850.

According to various embodiments, the wearable device may perform user-customized heart rate (HR) normalization in real-time, based on the heart rate (HR) trend (860) obtained through the IMU sensor 840 and the heart rate (HR) monitoring information (830) determined based on the biometric signal obtained by the PPG sensor 810 under the preset first condition in operation 870. According to various embodiments, the wearable device may derive normalization coefficients (880) based on the result of heart rate (HR) normalization.

According to various embodiments, the wearable device may perform heart rate trend $HR_{trend}$ normalization through the linear transform of Equation 1.

Equation 1

$$HR_{trend} \rightarrow a * HR_{trend} + b$$

Here, a is the deformation coefficient, and b is the translational coefficient.

The coefficient for time I may be obtained through minimization of Equation 2.

Equation 2

$$\min_{a_i, b_i} \sum_{j=i-n}^{j=i} Decay[j] * Mask[j] * (a_i * HR_{ACC}[j] + b_i - HR_{PPG}[j])^2$$

*Decay[j]* = $\gamma^{j-i}$, where $\gamma$ is the decay rate

Here, n is the window size for normalization. $HR_{PPG}$ is the measured heart rate, and "a*$HR_{ACC}$+b" is the normalized heart rate trend.

Referring to FIG. 8B, the wearable device may normalize the heart rate (HR) pattern by comparing the directly calculated heart rate (HR) with the heart rate (HR) pattern.

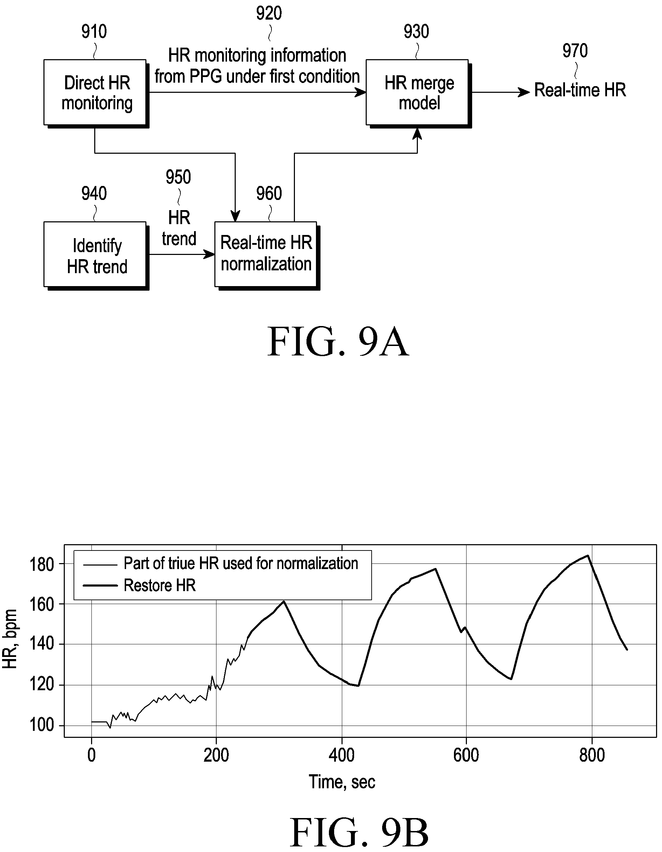

FIG. 9A is a view illustrating a heart rate (HR) modification process of a wearable device according to an embodiment of the disclosure. FIG. 9B is a view illustrating a modified heart rate (HR) according to an embodiment of the disclosure.

Referring to FIG. 9A, the wearable device may directly perform heart rate (HR) monitoring in operation 910 and provide heart rate (HR) monitoring information from the PPG sensor under the preset first condition to an HR merge model 930 in operation 920. According to various embodiments, the wearable device may identify the heart rate (HR) trend in operation 940 and, based on the heart rate (HR) trend in operation 950, perform real-time heart rate (HR) normalization in a user-customized manner in operation 960.

According to various embodiments, the wearable device may provide a real-time heart rate (HR) normalization result to the HR merge model 930. According to various embodiments, the wearable device may derive a real-time heart rate (HR) based on heart rate (HR) monitoring information from the preset PPG sensor under the preset first condition and the result of real-time heart rate (HR) normalization in operation 970. According to various embodiments, even when the quality of the heart rate measured by the PPG sensor is poor, the wearable device may estimate the real-time heart rate (HR) based on the real-time heart rate (HR) normalization result in operation 970.

According to various embodiments, the HR merge model 930 implemented in the wearable device may modify the heart rate (HR) by merging the normalized heart rate trend (a*HRACC + b) and the heart rate $HR_{PPG}$ measured using the quality metric (mask value) for time i as in Equation 3.

Equation 3

$$HR[i] = mask[i] * HR_{PPG}[i] +$$
$$(1 - mask[i]) * (a[i] * HR_{ACC}[i] + b[i])$$

Referring to FIG. 9B, the heart rate normalized under the first condition (when the measured signal quality is good) and the heart rate restored under the second condition (when the measured signal quality is poor) may be merged in real-time.

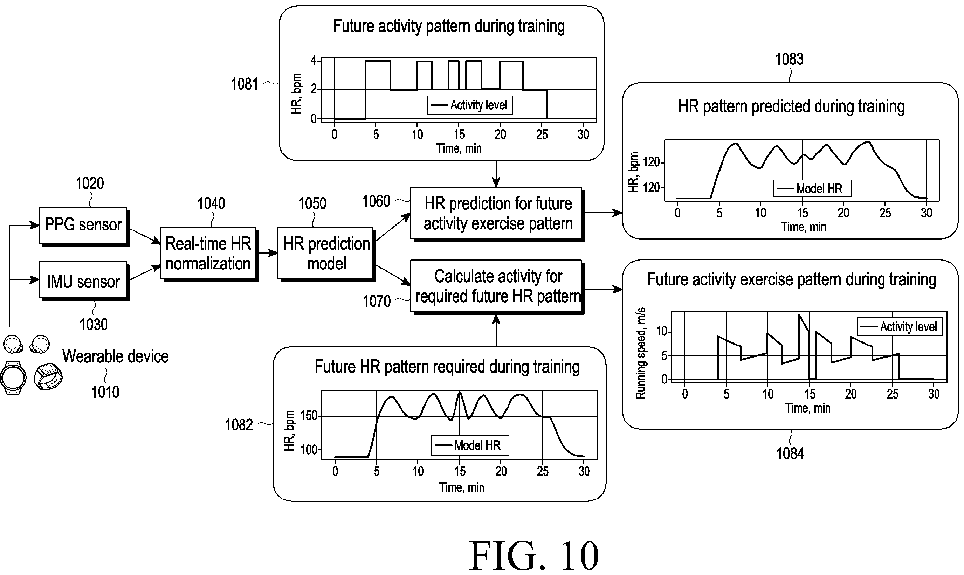

FIG. 10 is a view illustrating a heart rate (HR) prediction process of a wearable device according to an embodiment of the disclosure.

Referring to FIG. 10, at least one wearable device 1010 may real-time normalize the heart rate (HR) based on the user's activity information obtained from an IMU sensor 1030 and the user's biometric signal obtained from a PPG sensor 1020 in operation 1040.

According to various embodiments, the at least one wearable device 1010 may model the heart rate (HR) prediction based on the real-time heart rate (HR) normalization in operation 1050, predict the heart rate (HR) for the user's future activity exercise pattern based on the modeling result in operation 1060, and calculate the activity for the future heart rate (HR) pattern in operation 1070. According to various embodiments of the disclosure, the at least one wearable device 1010 may identify a heart rate (HR) pattern 1083 predicted during training based on a future activity pattern 1081 during the user's training. According to various embodiments, the at least one wearable device 1010 may identify a future activity exercise pattern 1084 during training based on a future heart rate (HR) pattern 1082 required during the user's training.

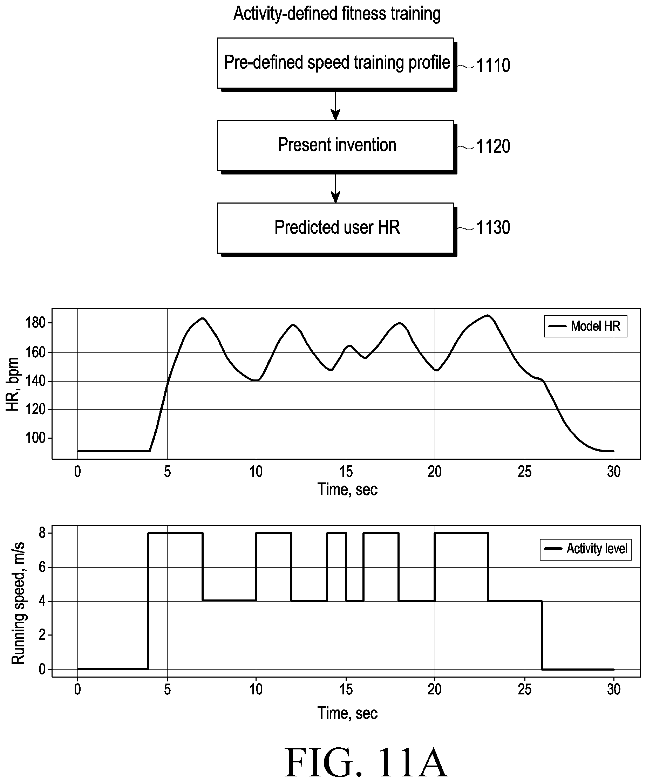

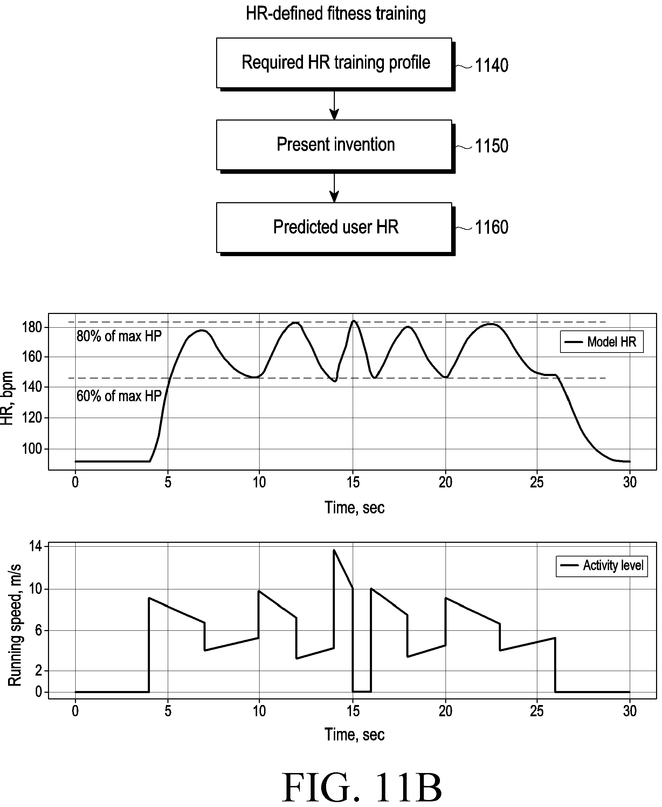

FIG. 11A is a view illustrating an activity defined fitness training according to an embodiment of the disclosure. FIG. 11B is a view illustrating a heart rate (HR) defined fitness training according to an embodiment of the disclosure.

Referring to FIG. 11A, if an embodiment 1120 of the present disclosure is applied to a predefined speed training profile 1110, the user heart rate (HR) may be predicted in operation 1130. According to various embodiments, the embodiment 1120 of the present disclosure may be any one of the embodiments described with reference to FIGS. 3 to 10. According to various embodiments, the wearable device may identify the user's activity level according to time during activity-defined fitness training and may perform heart rate (HR) modeling over time.

Referring to FIG. 11B, if an embodiment 1150 of the present disclosure is applied to a required heart rate (HR) training profile 1140, the user heart rate (HR) may be predicted in operation 1160. The embodiment 1150 of the present disclosure may be any one of the embodiments described with reference to FIGS. 3 to 10. According to various embodiments , the wearable device may identify the user's activity level according to time during heart rate (HR)-defined fitness training and may perform heart rate (HR) modeling over time.

According to various embodiments, the wearable device may recommend the remaining exercise based on the user's predicted heart rate (HR) and fatigue level. According to various embodiments, the wearable device may set a preliminary alarm when the user doing exercise is expected to reach a threshold heart rate (HR) in the future. According to various embodiments, the wearable device may recommend a next exercise to the user based on the required heart rate (HR) and the predicted heart rate (HR). According to various embodiments, the wearable device may recommend a long-term exercise profile in which the heart rate is recovered during exercise based on the required heart rate (HR) and the predicted heart rate (HR).

## Claims

1. An electronic device determining a vital sign while a user is active, the electronic device comprising:

   a first sensor for detecting a vital sign of a user;
   a second sensor for detecting an activity of the user; and
   at least one processor connected to the first sensor and the second sensor,
   wherein the at least one processor is configured to:

   identify the vital sign determined through a first signal obtained through the first sensor,
   normalize the vital sign to correspond to an activity characteristic of the user based on the vital sign determined through the first signal and an activity level of the user determined through a second signal obtained through the second sensor, and
   determine a merged vital sign by merging the vital sign determined through the first signal and a normalized vital sign.

2. The electronic device of claim 1, wherein the at least one processor is configured to:

   identify whether a quality value of the vital sign determined through the first signal is a threshold or more, and
   identify a first vital sign, of which the quality value is the threshold or more, among the vital sign determined through the first signal.

3. The electronic device of claim 2, wherein the at least one processor is configured to:

   identify the normalized vital sign, of which the quality value is less than the threshold, among the vital sign determined through the first signal, and
   determine the merged vital sign in real-time by merging the first vital sign and the normalized vital sign.

4. The electronic device of claim 1, wherein the at least one processor is configured to predict a vital sign for a future activity pattern of the user based on the vital sign determined through the first signal.

5. The electronic device of claim 1, wherein the at least one processor is configured to calculate a future activity level pattern of the user for a required vital sign pattern.

6. The electronic device of claim 1, wherein the at least one processor is configured to calculate or restore a vital sign trend based on the activity level, determined through the second signal obtained through the second sensor, and machine learning.

7. The electronic device of claim 1, wherein the at least one processor is configured to merge the vital sign determined through the first signal and the normalized vital sign based on machine learning.

8. The electronic device of claim 1,

   wherein the first sensor is at least one of a photoplethysmogram (PPG) sensor, an electrocardiogram (ECG) sensor, and a bioimpedance (BIA) sensor, and
   wherein the second sensor is at least one of a gyroscope, an accelerometer, a geomagnetic sensor, a radar sensor, and a video camera.

9. The electronic device of claim 1, wherein the vital sign is any one of a heart rate, a stress value, a respiration rate, and a lactate value.

10. The electronic device of claim 1, wherein the activity level is determined based on at least one of an activity type of the user, an activity intensity of the user, and an activity duration of the user.

11. A method for operating an electronic device determining a vital sign while a user is active, the method comprising:

identifying a vital sign of the user determined through a first signal obtained through a first sensor;
normalizing the vital sign based on an activity level of the user determined through a second signal obtained through a second sensor; and
modifying the vital sign by merging the vital sign determined through the first signal and a normalized vital sign.

12. The method of claim 11, further comprising:

identifying whether a quality value of the vital sign determined through the first signal is a threshold or more; and
identifying a first vital sign, of which the quality value is the threshold or more, among the vital sign determined through the first signal.

13. The method of claim 12, further comprising:

identifying the normalized vital sign, of which the quality value is less than the threshold, among the vital sign determined through the first signal; and
determining the vital sign in real-time by merging the first vital sign and the normalized vital sign.

14. The method of claim 11, further comprising:
predicting a vital sign for a future activity pattern of the user based on the modified vital sign.

15. The method of claim 11, further comprising:
calculating a future activity level pattern of the user for a required vital sign pattern.

FIG. 1

FIG. 2

300

/ 310

/ 320

| Wearable device | Server |
|---|---|

/ 312

/ 314

/ 322

First sensor

Second sensor

Communication module

/ 316

/ 318

/ 324

Processor

Communication module

Processor

# FIG. 3

**FIG. 4**

400

401 Wearable device

403 Direct sensor (PPG/ECG)

405 Direct HR & HR quality monitoring

407 HR merge model → Real-time HR

409 — Calculate real-time HR by PPG under second condition
- Good quality / Poor quality — PPG
- Directly measured HR / Current moment / Estimated HR — HR

HR monitoring information from PPG under first condition

411 IMU

413 Identify HR trend → HR trend

415 Real-time HR normalization

417 HR prediction model → Predicted HR

419 — HR vs activity prediction
- Activity level / Time
- Calculated HR / Current moment / Predicted HR / HR / Time

501
Biometric sensor

503
Activity sensor

505
1. Identify quality of
direct biometric signal

507
2. Identify direct biometric
signal of good quality

509
3. Identify trend pattern
from activity sensor

511
4. Normalize vital sign trend pattern

513
5. Restore vital sign at
moment of poor quality

515
6. Predict vital sign

517
7. Calculate future activity
for vital sign pattern

FIG. 5

When HR spectrum is not present in PPG spectrogram

When HR spectrum is present in PPG spectrogram

FIG. 6

FIG. 7

810

PPG sensor

820

Direct HR &
HR quality
monitoring

830

HR monitoring information
from PPG under first condition

840

IMU sensor

850

Identify
HR trend

860

HR
trend

870

Real-time HR
normalization

880

Normalization
coefficients

## FIG. 8A

a = 60.71   b = 95.97

Normalized estimated HR, bpm

Trend HR, a.u.

● Experimental poins of HR_true vs. HR_model

—— Regression: HR_true = a*HR_model + b

## FIG. 8B

910        920        930        970

Direct HR monitoring → HR monitoring information from PPG under first condition → HR merge model → Real-time HR

940        950        960

Identify HR trend → HR trend → Real-time HR normalization

FIG. 9A

FIG. 9B

FIG. 10

Activity-defined fitness training

FIG. 11A

HR-defined fitness training

Required HR training profile ~1140

↓

Present invention ~1150

↓

Predicted user HR ~1160

FIG. 11B

## INTERNATIONAL SEARCH REPORT

| International application No. |
|---|
| **PCT/KR2023/000577** |

**A. CLASSIFICATION OF SUBJECT MATTER**

**A61B 5/0205**(2006.01)i; **A61B 5/024**(2006.01)i; **A61B 5/11**(2006.01)i; **A61B 5/00**(2006.01)i; **A61B 5/28**(2021.01)i; **A61B 5/053**(2006.01)i; **G16H 20/30**(2018.01)i

According to International Patent Classification (IPC) or to both national classification and IPC

**B. FIELDS SEARCHED**

Minimum documentation searched (classification system followed by classification symbols)

A61B 5/0205(2006.01); A61B 5/00(2006.01); A61B 5/04(2006.01); A61B 5/08(2006.01); A61B 5/11(2006.01); G16H 40/67(2018.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 생체 사인 (vital sign), 활동 (activity), 레벨 (level), 정규화 (normalization), 머징 (merging), 예측 (prediction)

**C. DOCUMENTS CONSIDERED TO BE RELEVANT**

| Category* | Citation of document, with indication, where appropriate, of the relevant passages | Relevant to claim No. |
|---|---|---|
| Y | KR 10-2021-0034724 A (SAMSUNG ELECTRONICS CO., LTD.) 31 March 2021 (2021-03-31)<br>See paragraphs [0030]-[0071]; and claims 1, 5 and 9. | 1-15 |
| Y | JP 2018-516616 A (KONINKLIJKE PHILIPS N.V.) 28 June 2018 (2018-06-28)<br>See claim 2. | 1-15 |
| A | JP 6004152 B2 (SEIKO EPSON CORP.) 05 October 2016 (2016-10-05)<br>See entire document. | 1-15 |
| A | US 2021-0251573 A1 (KONINKLIJKE PHILIPS N.V.) 19 August 2021 (2021-08-19)<br>See entire document. | 1-15 |
| A | KR 10-2016-0144620 A (ROEMSYSTEM CORP.) 19 December 2016 (2016-12-19)<br>See entire document. | 1-15 |

| ☐ Further documents are listed in the continuation of Box C. | ☑ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **18 April 2023** | **19 April 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**
Information on patent family members

International application No.

**PCT/KR2023/000577**

| Patent document cited in search report | | | Publication date (day/month/year) | Patent family member(s) | | | Publication date (day/month/year) |
|---|---|---|---|---|---|---|---|
| KR | 10-2021-0034724 | A | 31 March 2021 | US | 2021-0085191 | A1 | 25 March 2021 |
| | | | | WO | 2021-054582 | A1 | 25 March 2021 |
| JP | 2018-516616 | A | 28 June 2018 | CN | 107529991 | A | 02 January 2018 |
| | | | | CN | 107529991 | B | 18 December 2020 |
| | | | | EP | 3282950 | A1 | 21 February 2018 |
| | | | | EP | 3282950 | B1 | 30 December 2020 |
| | | | | JP | 6770527 | B2 | 14 October 2020 |
| | | | | US | 11534130 | B2 | 27 December 2022 |
| | | | | US | 2018-0125444 | A1 | 10 May 2018 |
| | | | | WO | 2016-166318 | A1 | 20 October 2016 |
| JP | 6004152 | B2 | 05 October 2016 | JP | 2013-027550 | A | 07 February 2013 |
| US | 2021-0251573 | A1 | 19 August 2021 | CN | 112437629 | A | 02 March 2021 |
| | | | | EP | 3581093 | A1 | 18 December 2019 |
| | | | | EP | 3806713 | A1 | 21 April 2021 |
| | | | | JP | 2021-528135 | A | 21 October 2021 |
| | | | | WO | 2019-238525 | A1 | 19 December 2019 |
| KR | 10-2016-0144620 | A | 19 December 2016 | KR | 10-1739484 | B1 | 24 May 2017 |

Form PCT/ISA/210 (patent family annex) (July 2022)